# EUROPEAN PATENT SPECIFICATION

(11) **EP 4 289 470 B1**
(45) Date of publication and mention of the grant of the patent: **19.03.2025**
(21) Application number: 21924874.7
(22) Date of filing: 24.12.2021
(51) Int. Cl.: A61N 5/06

(54) **LIGHT-IRRADIATION-TYPE DEPILATION DEVICE**
LICHTBESTRAHLUNGS-ENTHAARUNGSVORRICHTUNG
DISPOSITIF D'ÉPILATION DE TYPE À IRRADIATION LUMINEUSE

(30) Priority: 05.02.2021 JP 2021017225
(43) Date of publication of application: 13.12.2023
(73) Proprietor: Panasonic Intellectual Property Management Co., Ltd., Kadoma-shi, Osaka 571-0057 (JP)
(72) Inventor: SAKAMOTO, Ryohei, Kadoma-shi, Osaka 571-0057 (JP); KASUGAI, Hideki, Kadoma-shi, Osaka 571-0057 (JP)
(74) Representative: Grünecker Patent- und Rechtsanwälte PartG mbB
(86) International application number: PCT/JP2021/048146
(87) International publication number: WO 2022/168489

(56) References cited:
- WO-A1-2011/037122
- JP-A- 2003 126 276
- JP-A- 2008 289 812
- JP-A- 2012 239 874
- JP-A- 2013 111 391
- JP-A- 2013 111 391
- US-A1- 2014 358 132

## Description

### TECHNICAL FIELD

The present disclosure relates to a light-emitting depilation device.

### BACKGROUND ART

Conventionally, a light-emitting depilation device that removes hair by irradiating the hair with light has been known. The light-emitting depilation device irradiates the skin surface of a user with light at a specific wavelength to promote hair removal by causing the light to act on melanin in hair follicles. As the light-emitting depilation device, for example, an apparatus such as that disclosed in Patent Literature 1 has been known.

Patent Literature 1 discloses a light-emitting depilation device including a light source that emits treatment light and detection light that are to be incident on an object, a photodetector that detects the detection light for detecting the object, and a control unit for controlling the light source. The control unit determines an absorption of the detection light from the detected detection light, and controls the light source so as to generate the treatment light based on the determined absorption. The treatment light has a wavelength within the range of 570 nm to 1200 nm, an energy density within the range of 2 J/cm² to 30 J/cm², and a pulse duration within 1 ms to 600 ms.

### Citation List

### Patent Literature

PTL 1: Japanese Patent No. 5715128
PTL 2: JP2013-111391 A

### SUMMARY OF THE INVENTION

The conventional light-emitting depilation device controls the application of light based on the characteristics of the object to be irradiated with the light. However, the conventional light-emitting depilation device might emit light even when the light-emitting depilation device is not fully in contact with the object. For example, the light may be emitted even when depilation device is in partial contact with the object, the partial contact being a condition in which a portion provided with the light detector is in contact with the object but another portion provided with the light source and on the opposite side of the light detector is separated from the object. In such a condition, a large amount of light may leak from the space between the light-emitting depilation device and the object, and the leaked light may get into the eyes of a user. In addition, the conventional light-emitting depilation device, which controls the application of light based on the characteristics of the object, may cause inflammation on the skin if the object is irradiated with treatment light having high irradiation energy.

The present disclosure provides a light-emitting depilation device capable of suppressing leakage of light or inflammation of the skin. The invention and its most advantageous embodiments are defined in the appended set of claims.

A light-emitting depilation device according to an aspect of the present disclosure includes a light source, a skin cooling unit, and a push switch. The light source emits light having a wavelength of 400 nm or longer and 1200 nm or shorter. The skin cooling unit faces the light source, transmits the light emitted from the light source, and cools skin upon coming into contact with the skin. The push switch includes a pressing unit surrounding an outer periphery of the light source and the skin cooling unit. When the pressing unit is not being pressed, the pressing unit is protruding from a surface of the skin cooling unit, the surface being a surface that is to be brought into contact with the skin, toward an opposite side of the light source with respect to the skin cooling unit. When the pressing unit is pressed, a surface pressed by the skin moves closer to the light source, with respect to the skin cooling unit. The push switch is configured to switch the light source to emit light and not to emit light to cause the light source to keep emitting the light at least for a part of time while the pressing unit is pressed, and not to emit the light while the pressing unit is not pressed.

According to the present disclosure, it is possible to achieve a light-emitting depilation device capable of suppressing leakage of light or inflammation of the skin.

### BRIEF DESCRIPTION OF THE DRAWINGS

Fig. 1 is a cross-sectional view illustrating a configuration of a light-emitting depilation device according to a present exemplary embodiment.
Fig. 2 is a cross-sectional view taken along line II-II in Fig. 1.
Fig. 3 is a perspective view illustrating an example of a schematic arrangement of light source according to the present exemplary embodiment.
Fig. 4 is a control block diagram related to a controller.
Fig. 5 is a cross-sectional view illustrating a configuration before the light-emitting depilation device is used.
Fig. 6 is a cross-sectional view illustrating a configuration before a push switch of the light-emitting depilation device is pressed.
Fig. 7 is a cross-sectional view illustrating a configuration after the push switch of the light-emitting depilation device is pressed.
Fig. 8 is a cross-sectional view illustrating a configuration while the skin is irradiated with the light of the light-emitting depilation device.

### DESCRIPTION OF EMBODIMENTS

An exemplary embodiment will now be explained in detail with reference to some drawings. Note that descriptions more in detail than necessary are sometimes omitted. For example, detailed descriptions of already well-known matters and redundant descriptions of substantially the same configurations may be omitted.

Note that the accompanying drawings and the following description are provided for those skilled in the art to fully understand the present disclosure, and are not intended to limit the subject matter described in the claims.

In the following exemplary embodiment, up-down direction Z is defined for light-emitting depilation device 1 by establishing a light outlet as upside, and the side facing the opposite side of the outlet as downside. Furthermore, in the explanation hereunder, one of horizontal directions extending along light-emitting depilation device 1 is defined as a width direction Y, and the direction orthogonal to up-down direction Z and width direction Y is defined as front-back direction X.

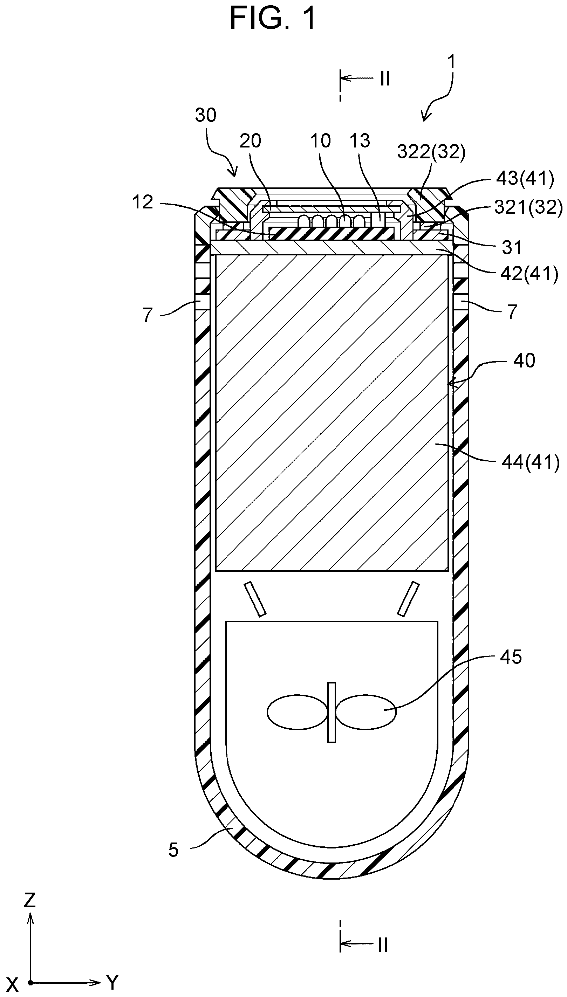

Light-emitting depilation device 1 according to the present exemplary embodiment will now be explained with reference to Figs. 1 to 8.

### [Configuration]

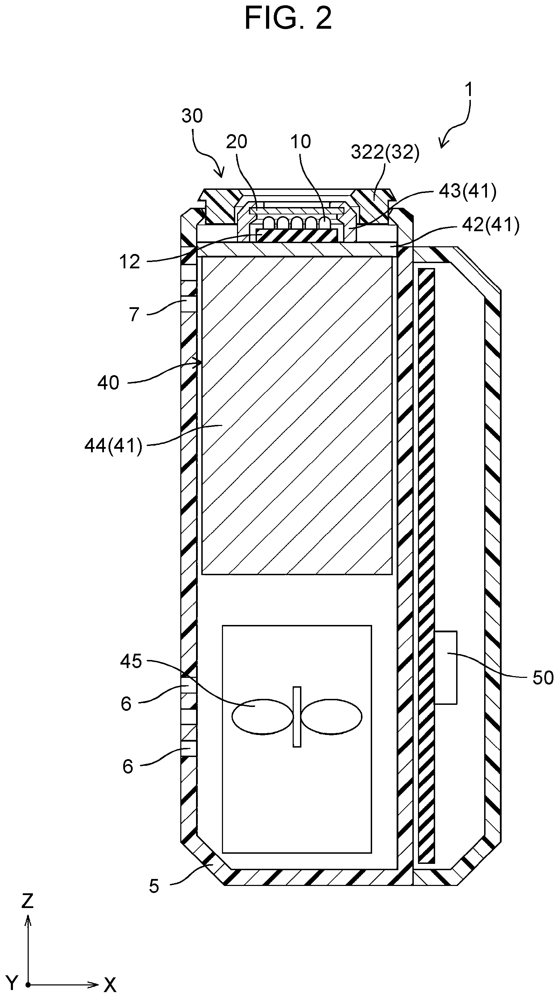

Fig. 1 is a cross-sectional view illustrating a configuration of light-emitting depilation device 1 according to the present exemplary embodiment, and Fig. 2 is a cross-sectional view taken along line II-II of Fig. 1. As illustrated in Figs. 1 and 2, light-emitting depilation device 1 includes housing 5, light source 10, temperature sensor 13, skin cooling unit 20, push switch 30, cooler 40, and controller 50.

One end of housing 5 has an opening serving as a light outlet of light-emitting depilation device 1. Light source 10 is provided inside the opening of housing 5, and emits light toward skin S of a person. Housing 5 has a bottom on the opposite side of light source 10. Housing 5 has a plurality of first openings 6 and a plurality of second openings 7. The outer air is collected through the plurality of first openings 6, and the air is discharged through the plurality of second openings 7. Light source 10, temperature sensor 13, skin cooling unit 20, push switch 30, cooler 40, and controller 50 are housed inside housing 5.

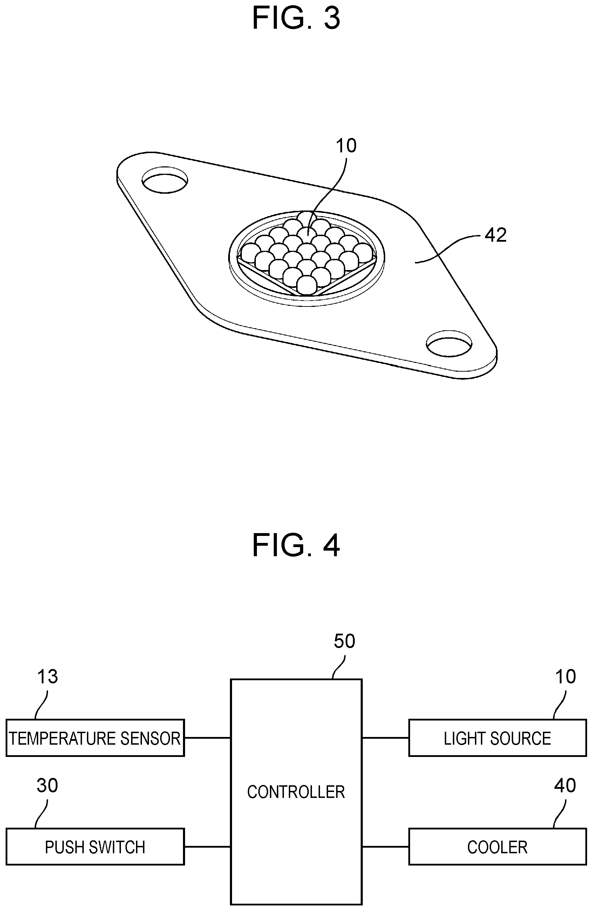

Fig. 3 is a perspective view illustrating an example of a schematic arrangement of light source 10 according to the present exemplary embodiment. In Fig. 3, some structures such as temperature sensor 13, skin cooling unit 20, push switch 30, and cooler 40 are omitted. As illustrated in Figs. 1 to 3, in the present exemplary embodiment, light source 10 includes a plurality of LEDs (light-emitting diodes). The LEDs are mounted on substrate 12 at substantially equal intervals therebetween. Light source 10 is electrically connected to a power supply (not illustrated). Light source 10 emits light by receiving the supply power from the power supply.

Light source 10 emits light having a wavelength of 400 nm or longer and 1200 nm or shorter. By irradiating skin S with the light as described above, melanin in hair follicles absorbs the light, and becomes heated. This heat damages the hair matrix in the hair follicle, and promotes removal of the hair. The wavelength of the light may be 500 nm or longer, 600 nm or longer, 700 nm or longer, or 800 nm or longer. The wavelength of the light may be 1000 nm or shorter or 900 nm or shorter. The light emitted from light source 10 may be light having a peak wavelength within a range between 400 nm or longer and 1200 nm or shorter. Even with the light having a peak wavelength within such a range, emitted light may include a wavelength component outside this range. In addition, all of the LEDs do not need to have the same wavelength spectrum, and LEDs emitting light at different wavelength spectra may be used in combination. The wavelength is a wavelength of light emitted from light source 10 at a temperature of 25°C.

Light source 10 preferably emits the light with an irradiance condition of 15 W/cm² or higher and 50 W/cm² or lower. By irradiating the hair with light at an irradiance of 15 W/cm² or higher, a high depilatory effect can be given to the hair during an initial growth period to a growth period. By emitting the light at an irradiance of 50 W/cm² or lower, it is possible to suppress an increase in the skin temperature due to the exposure to the light. Therefore, skin S is cooled more reliably, and irritations of the skin can be reduced. The irradiance may be 20 W/cm² or higher, 25 W/cm² or higher, or 30 W/cm² or higher. The irradiance may be 45 W/cm² or lower or 40 W/cm² or lower.

In the present exemplary embodiment, the light emitted from light source 10 is pulsed light that is intermittently emitted. Light source 10 preferably emits light intermittently with a condition of irradiation time of 500 ms or longer and 1000 ms or shorter. By exposing the hair to light for 500 ms or longer, a high depilatory effect can be given to the hair during an initial growth period to a growth period. In addition, by setting the exposure to the light to 1000 ms or shorter, it is possible to suppress an increase in the skin temperature due to the exposure to the light. Therefore, skin S is cooled more reliably, and irritations of the skin can be reduced. The irradiation time may be 600 ms or longer. The irradiation time may be 900 ms or shorter or 800 ms or shorter.

The energy of each pulse of the light emitted from light source 10 is preferably 9 J/cm² or higher and 50 J/cm² or lower. By setting the energy to 9 J/cm² or higher, a high depilatory effect can be achieved. In addition, in light-emitting depilation device 1 including skin cooling unit 20, by setting the energy to 50 J/cm² or lower, it is possible to suppress an increase in the skin temperature due to the exposure to the light. Therefore, skin S is cooled more reliably, and irritations of the skin can be reduced.

Skin cooling unit 20 is disposed at a position facing light source 10. Skin cooling unit 20 may be in contact with light source 10, or may be disposed spaced from light source 10. Skin cooling unit 20 is provided in a manner coming into contact with skin S on the side opposite to light source 10. Skin cooling unit 20 is made of a translucent material. When light is emitted from light source 10, skin cooling unit 20 transmits the light emitted from light source 10, and skin S is irradiated with the light having passed through skin cooling unit 20. Skin cooling unit 20 is, for example, a translucent plate, and in the present exemplary embodiment, disk-shaped skin cooling unit 20 is used.

Skin cooling unit 20 is preferably made of a material that does not absorb much of the light emitted from light source 10. Specifically, the total light transmittance of skin cooling unit 20 is preferably 80% or higher. By setting the total light transmittance to 80% or higher, skin cooling unit 20 can transmit most of the light emitted from light source 10. Therefore, a large amount of light can be delivered to the melanin, and the depilatory effect can be promoted. In addition, because the amount of light absorbed and converted into heat by skin cooling unit 20 can be reduced, a temperature rise in skin cooling unit 20 can be suppressed. From the viewpoint of suppressing absorption of the light by skin cooling unit 20, the total light transmittance is preferably 90% or higher, still more preferably 95% or higher, and particularly preferably 99% or higher. The upper bound of the total light transmittance is 100%. The total light transmittance can be measured according to JIS K7361-1:1997.

The refractive index of skin cooling unit 20 is preferably 1.7 or higher. By setting the refractive index of skin cooling unit 20 to 1.7 or higher, skin cooling unit 20 does not absorb much of the light emitted from light source 10. When the refractive index is higher, skin cooling unit 20 transmits a greater amount of light. Therefore, the refractive index is more preferably 1.8 or higher, still more preferably 1.9 or higher, and particularly preferably 2.0 or higher. The upper bound of the refractive index is not particularly limited, but may be 10. The refractive index can be measured by a minimum deviation method according to JIS B7071-1:2015.

Skin cooling unit 20 cools skin S upon coming into contact with skin S. Skin cooling unit 20 preferably includes a material having high thermal conductivity. The thermal conductivity of skin cooling unit 20 is preferably 1 W/mK or higher. By setting the thermal conductivity to 1 W/mK or higher, even when skin cooling unit 20 becomes heated by the light from light source 10 and skin S, the heat is dissipated easily. Therefore, skin S can be cooled effectively. The cooling effect of skin cooling unit 20 tends to increase by setting the thermal conductivity higher, because skin cooling unit 20 becomes more thermally conductive. Therefore, from the viewpoint of cooling efficiency, the thermal conductivity of skin cooling unit 20 is more preferably 2 W/mK or higher, still more preferably 10 W/mK or higher, particularly preferably 30 W/mK or higher, and most preferably 100 W/mK or higher. The upper bound of the thermal conductivity is not particularly limited, but may be 100,000 W/mK. The thermal conductivity can be measured by a laser flash method according to JIS R1611:2010.

Skin cooling unit 20 may contain an inorganic substance. Specifically, skin cooling unit 20 preferably includes at least one selected from the group consisting of Al₂O₃, ZnO, ZrO₂, MgO, GaN, AlN, and diamond. Because these materials have a high refractive index and a high thermal conductivity, translucency and thermal conductivity of skin cooling unit 20 can be improved. Al₂O₃ (sapphire) has a refractive index of 1.79 and a thermal conductivity of 42 W/mK. ZnO has a refractive index of 2.01 and a thermal conductivity of 20 W/mK. ZrO₂ has a refractive index of 2.13 and a thermal conductivity of 3 W/mK. MgO has a refractive index of 1.74 and a thermal conductivity of 47 W/mK. GaN has a refractive index of 2.346 and a thermal conductivity of 200 W/mK. AlN has a refractive index of 2.175 and a thermal conductivity of 150 W/mK. Diamond has a refractive index of 2.417 and a thermal conductivity of 1000 W/mK.

Skin cooling unit 20 may contain resin such as a silicone resin, from the viewpoint of heat resistance and translucency. Skin cooling unit 20 may also include resin such as silicone resin, and highly thermal conductive filler that is dispersed in the resin. By including such highly thermal conductive filler in skin cooling unit 20, the heat of skin cooling unit 20 is dissipated more quickly, so that skin S can be cooled effectively. The highly thermally conductive filler may contain an inorganic substance such as those listed above.

The proportion of the inorganic substance in skin cooling unit 20 is preferably 10 mass% or higher. By setting the proportion of the inorganic substance in skin cooling unit 20 to 10 mass% or higher, the thermal conductivity of skin cooling unit 20 can be improved. The proportion of the inorganic substance in skin cooling unit 20 is more preferably 30 mass% or higher, still more preferably 50 mass% or higher, particularly preferably 70 mass% or higher, and most preferably 90 mass% or higher.

Skin cooling unit 20 is preferably cooled to -5°C or higher and 35°C or lower. By cooling skin cooling unit 20 to -5°C or higher, it is possible to cool skin S without causing much pain in skin S by cooling. By cooling skin cooling unit 20 to 35°C or lower, it is possible to suppress inflammation due to a rise in the skin temperature due to the exposure to the irradiation. Skin cooling unit 20 is more preferably cooled to 0°C or higher, still more preferably 5°C or higher, and particularly preferably 10°C or higher. In addition, skin cooling unit 20 is more preferably cooled to 30°C or lower, still more preferably 25°C or lower, particularly preferably 20°C or lower, and most preferably 15°C or lower.

Temperature sensor 13 detects the temperature of skin cooling unit 20. By detecting the temperature of skin cooling unit 20, the temperature of skin cooling unit 20 can be controlled precisely. Temperature sensor 13 is provided in a manner facing skin cooling unit 20. Specifically, temperature sensor 13 is provided on substrate 12. In the present exemplary embodiment, temperature sensor 13 includes a contact temperature sensor. Examples of the contact type temperature sensor include a thermistor, a thermocouple, and a resistance thermometer bulb.

Push switch 30 is a momentary switch. Push switch 30 is provided on connecting part 42 of cooler 40. In front-back direction X and width direction Y, push switch 30 is disposed outer side of light source 10 and skin cooling unit 20, and is provided in a manner surrounding light source 10 and skin cooling unit 20. Push switch 30 includes two base portions 31 and pressing unit 32.

Two base portions 31 are fixed on connecting part 42, on the outer side of holder 43 of cooler 40 in such a manner that light source 10 and skin cooling unit 20 are disposed between base portions 31 in width direction Y. Each base portion 31 has a quadrangular prism shape extending upward from connecting part 42.

Pressing unit 32 is engaged with base portions 31, and moves in the up-down directions Z by being pressed by skin S. Pressing unit 32 covers the outer periphery of light source 10 and skin cooling unit 20. Pressing unit 32 includes two first components 321 and one second component 322.

Each first component 321 has a columnar shape extending upward in up-down direction Z from corresponding base portion 31, and is provided at substantially center in front-back direction X and width direction Y of corresponding base portion 31. Each second component 322 is disposed so as to be in contact with a surface of first component 321, the surface facing opposite side of base portion 31. Second component 322 has a through-hole at the center in front-back direction X and width direction Y, and has a donut-like shape extending in up-down direction Z. Light source 10 and skin cooling unit 20 are disposed inside the through-hole of second component 322. A part of a surface of second component 322 protrudes upward in up-down direction Z from the surface of skin cooling unit 20, the surface facing on the opposite side of light source 10. In the present exemplary embodiment, first component 321 and second component 322 are different components that are separated from each other, but pressing unit 32 may be one component that is integrally and continuously formed. In addition, the numbers of base portions 31, first components 321, and second components 322 are not particularly limited, and may be changed as appropriate.

When pressing unit 32 is not being pressed, pressing unit 32 is protruding from a surface of skin cooling unit 20, the surface being a surface to be brought into contact with the skin S, in the direction opposite to light source 10 with respect to skin cooling unit 20 (upward direction in up-down direction Z). Each of base portion 31 and pressing unit 32 has an internal contact, not illustrated. Push switch 30 is configured in such a manner that, while pressing unit 32 is not pressed, the contact inside base portion 31 is not brought into contact with the contact inside pressing unit 32, and open the circuit to which light source 10 is connected. When pressing unit 32 is pressed, the surface thereof being pressed by skin S moves toward light source 10 (downward in up-down direction Z), with respect to skin cooling unit 20. Therefore, the contact provided inside base portion 31 is brought into contact with the contact provided inside pressing unit 32, and close the circuit to which light source 10 is connected.

An elastic body, not illustrated, is provided between base portions 31 and pressing unit 32. When pressing unit 32 is pressed, the elastic body becomes elastically deformed, and pushes back pressing unit 32 by the elastic force generated by the elastic deformation. Therefore, when the force pressing pressing unit 32 is removed, the elastic body acts on pressing unit 32 and brings pressing unit 32 back to the original position, and the surface of pressing unit 32 that is in contact with skin S moves in a direction separating from base portion 31 (upward in up-down direction Z).

Push switch 30 switches to cause light source 10 to emit light and not to emit light in such a manner that light source 10 emits light during at least some of the time while pressing unit 32 is pressed, and light source 10 does not to emit light while pressing unit 32 is not pressed. This configuration, therefore, enables skin S to be irradiated with light during at least some of the time while light-emitting depilation device 1 is pressed against skin S, and stops the light emission when light-emitting depilation device 1 is separated from skin S.

The light emission may be started immediately after push switch 30 is pressed, or may be started a predetermined time elapses from when push switch 30 is pressed. The timing at which light source 10 starts the light emission may be controlled by controller 50. Light-emitting depilation device 1 may also cause light source 10 to emit light after skin S comes into contact with the surface of skin cooling unit 20. In this manner, skin surface having been cooled is irradiated with light. In this manner, heating of skin S is suppressed, so that it is possible to suppress irritation of skin S. In addition, because skin S is irradiated with light while held in contact with skin cooling unit 20, unevenness in the irradiation can be reduced, so that stable depilatory effect can be achieved.

Cooler 40 cools skin cooling unit 20. Because light-emitting depilation device 1 includes cooler 40, the temperature of skin cooling unit 20 can be further reduced. Therefore, the skin cooling effect by skin cooling unit 20 can be further improved. Cooler 40 includes heat sink 41 and air blower 45.

Heat sink 41 is connected to skin cooling unit 20, and dissipates the heat skin cooling unit 20 is deprived of. Heat sink 41 includes connecting part 42, holder 43, and heat sink fins 44.

Connecting part 42 is a plate-like member. On a first surface that is one surface of the connecting part 42, substrate 12 is provided. Substrate 12 is smaller than connecting part 42, and is provided to fit inside connecting part 42. Holder 43 and push switch 30 are connected to the outer side of substrate 12, on the first surface of connecting part 42. Heat sink fins 44 are provided on a second surface, that is the surface on the opposite side of the first surface of connecting part 42.

Holder 43 protrudes upward in up-down direction Z from the first surface of connecting part 42, and holds the entire periphery of skin cooling unit 20. Therefore, light source 10 is covered by skin cooling unit 20, holder 43, and connecting part 42. The heat generated by light source 10 is dissipated via skin cooling unit 20 and heat sink 41 of cooler 40. While holder 43 holds the entire periphery of skin cooling unit 20, holder 43 may be connected to at least a part of skin cooling unit 20.

Heat sink fins 44 are provided on the second surface of connecting part 42 (surface facing opposite side of light source 10). Therefore, holder 43 and connecting part 42 transfers the heat from skin cooling unit 20 to heat sink fins 44. Heat sink fins 44 include a plurality of fins, and has a large contact area with air, so that heat is dissipated quickly.

Heat sink 41 preferably contains material having excellent thermal conductivity. The thermal conductivity of heat sink 41 may be higher than that of skin cooling unit 20. Specifically, heat sink 41 may contain metal such as aluminum, iron, or copper. Holder 43, connecting part 42, and heat sink fins 44 may be made of the same material or may be made of different materials.

Air blower 45 cools heat sink 41 by sending the air to heat sink 41. Air blower 45 includes, for example, a fan, and when the fan is rotated, an air flow is generated. Housing 5 has a plurality of first openings 6 at positions facing air blower 45. Housing 5 has a plurality of second openings 7 at positions facing heat sink fins 44. Therefore, when air blower 45 is driven, the air collected from the outside of housing 5 through the plurality of first openings 6 is sent to heat sink fins 44. The heat of the air having been brought into contact with heat sink fins 44 is exchanged with the heat of the heat sink fins 44, and heat sink fins 44 are cooled thereby. The air heated by coming into contact with heat sink fins 44 is released outside of housing 5 via the plurality of second openings 7.

Explained in the present exemplary embodiment is an example in which light-emitting depilation device 1 uses air cooler 40 to cool skin cooling unit 20, but a Peltier element or the like may also be used to cool skin cooling unit 20, in addition to air cooler 40, or instead of air cooler 40. When a Peltier element is used to cool skin cooling unit 20, light-emitting depilation device 1 can cool skin cooling unit 20 much more intensely.

Fig. 4 is a control block diagram related to controller 50. Controller 50 controls light source 10 to emit light and not to emit light. Controller 50 controls to drive and to stop air blower 45. As illustrated in Fig. 4, temperature sensor 13 and push switch 30 are connected on the input side of controller 50. On the output side of controller 50, light source 10 and cooler 40 are connected. Controller 50 includes a computer system that includes a central processing unit (CPU), a read-only memory (ROM), and a random access memory (RAM). When the CPU executes a program stored in the ROM, the computer system functions as controller 50. In the example explained herein, the program executed by the CPU is recorded on the ROM included in the computer system in advance, but may also be provided by being recorded in a non-transitory recording medium such as a memory card, or be provided over a telecommunication line such as the Internet.

Controller 50 keeps light source 10 on or blinking while push switch 30 is being pressed. Controller 50 may cause light source 10 to emit light at the same time as push switch 30 is pressed, or may cause light source 10 to emit light after a predetermined time elapses from when push switch 30 is pressed.

Controller 50 may also be configured to cause cooler 40 to cool skin cooling unit 20 so as to bring the temperature of skin cooling unit 20 to -5°C or higher and 35°C or lower. Controller 50 may also be configured to receive a signal related to the temperature of skin cooling unit 20 from temperature sensor 13, and to drive cooler 40 to cool skin cooling unit 20 based on the signal. Controller 50 may also be configured to cool skin cooling unit 20 by controlling the output of air blower 45, for example. Furthermore, controller 50 may also be configured to cool skin cooling unit 20 by controlling the output of the Peltier element, for example. Specifically, controller 50 may drive the Peltier element when the temperature of skin cooling unit 20 becomes 35°C or higher, and may stop driving the Peltier element when the temperature of skin cooling unit 20 drops to a level lower than -5°C.

### [Operation]

An action and effects achieved by light-emitting depilation device 1 having the configuration described above will be explained below.

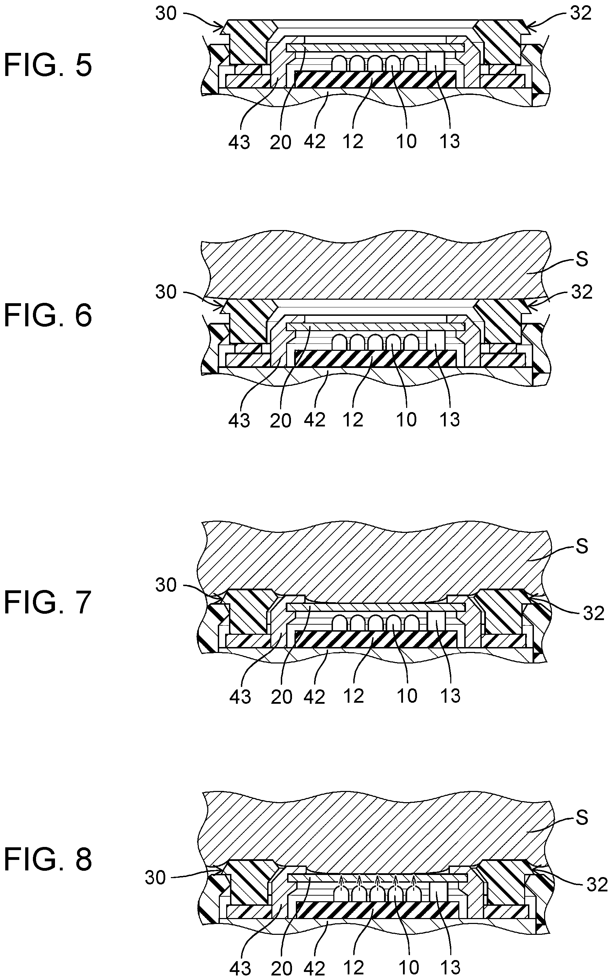

It will be now explained, with reference to Figs. 5 to 8, how light-emitting depilation device 1 emits light. Fig. 5 is a cross-sectional view illustrating a configuration before light-emitting depilation device 1 is used. Fig. 6 is a cross-sectional view illustrating a configuration before push switch 30 of light-emitting depilation device 1 is pressed. Fig. 7 is a cross-sectional view illustrating a configuration after push switch 30 of light-emitting depilation device 1 is pressed. Fig. 8 is a cross-sectional view illustrating a configuration while skin S is exposed to the light of light-emitting depilation device 1.

As illustrated in Fig. 5, before light-emitting depilation device 1 is used, push switch 30 remains unpressed. Therefore, pressing unit 32 of push switch 30 is protruding from the surface of skin cooling unit 20 that is to be brought into contact with skin S, toward the opposite side of light source 10 with respect to skin cooling unit 20. In this condition, light source 10 is not emitting light.

As illustrated in Fig. 6, during the use of light-emitting depilation device 1, light-emitting depilation device 1 is pressed against skin S of a user. Pressing unit 32 of push switch 30 is protruding from the skin contact surface of skin cooling unit 20. Therefore, push switch 30 first comes into contact with skin S of the user, and light source 10 becomes covered by skin S and push switch 30.

As illustrated in Fig. 7, push switch 30 is pressed by coming into contact with skin S. Specifically, when pressing unit 32 of push switch 30 is pressed, the surface being pressed by skin S moves closer to light source 10, with respect to skin cooling unit 20. Skin cooling unit 20 therefore then comes into contact with skin S, with light source 10 covered by skin S and push switch 30. Skin cooling unit 20 then becomes sealed by skin S. By being brought into contact with skin cooling unit 20, skin S is cooled.

As illustrated in Fig. 8, push switch 30 closes the circuit to which light source 10 is connected, and cause the light source 10 to emit light. Because skin cooling unit 20 is sealed by skin S, and light source 10 is also covered by push switch 30, skin S is irradiated with the light emitted from light source 10 with no leakage. In order to bring skin cooling unit 20 and skin S into contact with each other more reliably, light source 10 may be caused to emit light after a predetermined time elapses from when pressing unit 32 of push switch 30 is pressed.

### [Effects]

As described above, light-emitting depilation device 1 according to the present exemplary embodiment includes light source 10, skin cooling unit 20, and push switch 30. Light source 10 emits light having a wavelength of 400 nm or longer and 1200 nm or shorter. Skin cooling unit 20 is positioned facing light source 10, transmits the light emitted from light source 10, and cools skin S upon coming into contact with skin S. Push switch 30 includes pressing unit 32 surrounding the outer periphery of light source 10 and the skin cooling unit 20. When pressing unit 32 is not being pressed, pressing unit 32 is protruding from the surface of skin cooling unit 20, the surface being a surface that is to be brought into contact with skin S, toward the opposite side of light source 10 with respect to skin cooling unit 20. When pressing unit 32 is pressed, its surface being pressed by the skin S moves closer to light source 10, with respect to skin cooling unit 20. Push switch 30 switches to cause light source 10 to emit light and not to emit light in such a manner that light source 10 emits light during at least a part of the time while pressing unit 32 is pressed, and light source 10 does not to emit light while pressing unit 32 is not pressed.

Thus, light-emitting depilation device 1 can irradiate skin S with the light, with light source 10 covered by push switch 30 and skin S. Therefore, light-emitting depilation device 1 can suppress leakage of light. Light-emitting depilation device 1 can also cause skin cooling unit 20 to cool skin S by coming into contact with skin S at the time of light emission. Therefore, light-emitting depilation device 1 can suppress inflammation of skin S.

Light-emitting depilation device 1 may be light-emitting depilation device 1 including the skin cooling unit (skin cooling unit 20), in which the LED (light source 10) emits light after the top surface of the skin cooling unit (skin cooling unit 20) is pushed against the skin (skin S) and comes into contact with the skin (skin S). Even with such light-emitting depilation device 1, it is possible to suppress leakage of light or inflammation of skin S.

As light-emitting depilation device 1 according to the present exemplary embodiment, light source 10 may emit light after surface of skin S comes into contact with skin cooling unit 20.

In this manner, light-emitting depilation device 1 irradiates the skin S with the light while the surface of skin S is being cooled. The temperature rise of skin S is thus suppressed more reliably, so that light-emitting depilation device 1 can alleviate irritation of skin S. In addition, because skin S is irradiated with light while skin cooling unit 20 is held in contact with skin S, light-emitting depilation device 1 can suppress uneven irradiation of light, and achieve the depilatory effect stably.

As in light-emitting depilation device 1 according to the present exemplary embodiment, light source 10 may include an LED.

By using the LED as light source 10, the height of light-emitting depilation device 1 can be reduced, so that light-emitting depilation device 1 can be downsized. In addition, because LEDs are generally small, the space between the LED and skin cooling unit 20 in light-emitting depilation device 1 can be reduced. Therefore, light-emitting depilation device 1 can dissipate the heat generated by the LED not only via connecting part 42 of skin cooling unit 20 but also via skin cooling unit 20. Therefore, because light-emitting depilation device 1 can deplete the heat generated by the LED from around the LED, the LED can be cooled effectively.

As in light-emitting depilation device 1 according to the present exemplary embodiment, skin cooling unit 20 may be cooled to -5°C or higher and 35°C or lower.

As a result, light-emitting depilation device 1 can cool skin S without causing much pain in skin S by cooling, and can suppress inflammation due to a rise in the skin temperature due to the exposure to the irradiation.

As in light-emitting depilation device 1 according to the present exemplary embodiment, the total light transmittance of skin cooling unit 20 may be 80% or higher.

In this manner, skin cooling unit 20 can transmit most of the light emitted from light source 10. Therefore, light-emitting depilation device 1 can deliver a large amount of light to the melanin, and can promote the depilatory effect. In addition, because light-emitting depilation device 1 can reduce the amount of light absorbed and converted into heat by skin cooling unit 20, a temperature rise in skin cooling unit 20 can be suppressed.

As in light-emitting depilation device 1 according to the present exemplary embodiment, skin cooling unit 20 may include at least one selected from the group consisting of Al₂O₃, ZnO, ZrO₂, MgO, GaN, AlN, and diamond.

In this manner, light-emitting depilation device 1 can improve translucency and thermal conductivity of skin cooling unit 20. Therefore, light-emitting depilation device 1 can promote the depilatory effect, and enhance the cooling effect of skin cooling unit 20.

As in light-emitting depilation device 1 according to the present exemplary embodiment, light source 10 may emit light intermittently with a conditions of irradiance of 15 W/cm² or higher and 50 W/cm² and irradiation time of 500 ms or longer and 1000 ms or shorter.

In this manner, light-emitting depilation device 1 can achieve high depilatory effect on hair during an initial growth period to a growth period. In addition, light-emitting depilation device 1 can cool skin S more reliably, and can reduce irritation of the skin.

### (Other exemplary embodiments)

As described above, the above exemplary embodiment has been described as an example of the technology in the present disclosure. However, the technique in the present disclosure is not limited thereto, and can also be applied to exemplary embodiments in which changes, replacements, additions, omissions, and the like are made. Therefore, other exemplary embodiments will be described below.

Light-emitting depilation device 1 according to the above exemplary embodiment has been described to include light source 10 that includes an LED, as an example. However, light source 10 only needs to be able to emit light having a wavelength of 400 nm or longer and 1200 nm or shorter. Light source 10 is not limited to an LED, and may include, for example, a xenon lamp, a laser diode, and a combination thereof. With the use of an LED as light source 10, however, the size of light-emitting depilation device 1 can be reduced, as mentioned above.

Skin cooling unit 20 may include a wavelength cut filter that suppresses transmission of a specific wavelength. The wavelength cut filter may be provided, for example, on a surface of skin cooling unit 20 on the side facing light source 10 or the surface facing the opposite side of light source 10. In particular, when a xenon lamp is used as light source 10, light at various wavelengths is emitted, Therefore, by cutting light having a specific wavelength, for example, it is possible to suppress pain in skin S. Furthermore, by enabling to the light at a specific wavelength to be taken out, light-emitting depilation device 1 including skin cooling unit 20 that includes the wavelength cut filter can also improve the depilatory effect.

Skin cooling unit 20 may also include an antireflection film for preventing reflection of the light emitted from light source 10. The antireflection film may be provided on a surface of skin cooling unit 20 on the side facing light source 10, for example. By providing such an antireflection film to skin cooling unit 20, reflection of light is suppressed, and light-emitting depilation device 1 including skin cooling unit 20 including the antireflection film can irradiate skin S with a larger amount of light.

In addition, light-emitting depilation device 1 according to the above exemplary embodiment has been explained to cool skin cooling unit 20 using cooler 40, as an example. However, when the thermal conductivity of skin cooling unit 20 is high, it is not always necessary to cool skin cooling unit 20 using cooler 40, because the heat dissipation of skin cooling unit 20 is high.

Light-emitting depilation device 1 according to the above exemplary embodiment is explained to include cooler 40 that is connected to skin cooling unit 20 to cool skin cooling unit 20, as an example. However, cooler 40 does not need to be connected to skin cooling unit 20.

Note that, because the above-described exemplary embodiments are intended to illustrate the technique in the present disclosure, various changes, replacements, additions, omissions, and the like can be made within the scope of the claims and equivalents thereof.

### INDUSTRIAL APPLICABILITY

As described above, the light-emitting depilation device according to the present disclosure can be applied to a light-emitting depilation devices for business use and home use, for example.

### REFERENCE MARKS IN THE DRAWINGS

- 1: light-emitting depilation device
- 5: housing
- 6: first opening
- 7: second opening
- 10: light source
- 12: substrate
- 13: temperature sensor
- 20: skin cooling unit
- 30: push switch
- 31: base portion
- 32: pressing unit
- 40: cooler
- 41: heat sink
- 42: connecting part
- 43: holder
- 44: heat sink fins
- 45: air blower
- 50: controller
- 321: first component
- 322: second component
- S: skin

## Claims

1. A light-emitting depilation device comprising:
a light source (10) configured to emit light having a wavelength of 400 nm or longer and 1200 nm or shorter;
a skin cooling unit (20) that faces the light source, is configured to transmit the light emitted from the light source, and to cool
skin upon coming into contact with the skin; and
a push switch (30) that includes a pressing unit (32) surrounding an outer periphery of the light source and the skin cooling unit, wherein
when the pressing unit is not being pressed, the pressing unit protrudes from a surface of the skin cooling unit toward an opposite side of the light source with respect to the skin cooling unit, the surface of the skin cooling unit being a surface that is to be brought into contact with the skin,
when the pressing unit (32) is pressed by the skin, a surface of the pressing unit is pressed by the skin to be moved toward the light source with respect to the skin cooling unit, and
the push switch switches the light source to emit the light and not to emit the light to cause the light source to keep emitting the light at least for a part of time while the pressing unit is pressed, and not to emit the light while the pressing unit is not pressed.

2. The light-emitting depilation device according to Claim 1, configured to irradiate skin with light from the light source after the skin comes into contact with the surface of the skin cooling unit.

3. The light-emitting depilation device according to Claim 1 or 2, wherein the light source includes a light-emitting diode (LED).

4. The light-emitting depilation device according to any one of Claims 1 to 3, wherein the skin cooling unit is configured to be cooled to -5°C or higher and 35°C or lower.

5. The light-emitting depilation device according to any one of Claims 1 to 4, wherein a total light transmittance of the skin cooling unit is 80% or higher.

6. The light-emitting depilation device according to any one of Claims 1 to 5, wherein the skin cooling unit includes at least one selected from the group consisting of Al₂O₃, ZnO, ZrO₂, MgO, GaN, AlN, and diamond.

7. The light-emitting depilation device according to any one of Claims 1 to 6, wherein the light source is configured to emit light intermittently under conditions of an irradiance of 15 W/cm² or higher and 50 W/cm² or lower and an irradiation time of 500 ms or longer and 1000 ms or shorter.

## Patentansprüche

1. Lichtemittierendes Enthaarungsgerät, mit:
einer Lichtquelle (10), die ausgebildet ist, Licht mit einer Wellenlänge von 400 nm oder länger und 1200 nm oder kürzer zu emittieren;
einer Hautkühleinheit (20), die der Lichtquelle zugewandt und ausgebildet ist, das von der Lichtquelle emittierte Licht durchzulassen und bei Kontakt mit der Haut die Haut zu kühlen; und
einem Druckschalter (30), der eine Druckeinheit (32) aufweist, die einen äußeren Umfang der Lichtquelle und der Hautkühleinheit umgibt, wobei
die Druckeinheit, wenn sie nicht gedrückt wird, von einer Oberfläche der Hautkühleinheit in Richtung einer in Bezug auf die Hautkühleinheit gegenüberliegenden Seite der Lichtquelle vorsteht, wobei die Oberfläche der Haut eine Oberfläche ist, die mit der Haut in Kontakt zu bringen ist,
wenn die Druckeinheit (32) von der Haut gedrückt wird, eine Oberfläche der Druckeinheit, von der Haut gedrückt wird, um in Bezug auf die Hautkühleinheit in Richtung der Lichtquelle bewegt zu werden, und
der Druckschalter die Lichtquelle so schaltet, dass sie Licht emittiert und kein Licht emittiert, um zu bewirken, dass die Lichtquelle das Licht zumindest für einen Teil der Zeit emittiert wird, während die Drückeinheit gedrückt wird, und kein Licht emittiert, während die Druckeinheit nicht gedrückt wird.

2. Lichtemittierendes Enthaarungsgerät gemäß Anspruch 1, das ausgebildet ist, die Haut mit Licht von der Lichtquelle zu bestrahlen, nachdem die Haut mit der Oberfläche der Hautkühleinheit in Kontakt gekommen ist.

3. Lichtemittierendes Enthaarungsgerät gemäß Anspruch 1 oder 2, wobei die Lichtquelle eine lichtemittierende Diode (LED) aufweist.

4. Lichtemittierendes Enthaarungsgerät gemäß einem der Ansprüche 1 bis 3, wobei die Hautkühleinheit ausgebildet ist, auf -5 °C oder höher und 35 °C oder niedriger gekühlt zu werden.

5. Lichtemittierendes Enthaarungsgerät gemäß einem der Ansprüche 1 bis 4, wobei die Gesamtlichtdurchlässigkeit der Hautkühleinheit 80 % oder höher ist.

6. Lichtemittierendes Enthaarungsgerät gemäß einem der Ansprüche 1 bis 5, wobei die Hautkühleinheit Al₂O₃ und/oder ZnO und/oder ZrO₂ und/oder MgO und/oder GaN und/oder AIN und/oder Diamant aufweist.

7. Lichtemittierendes Enthaarungsgerät gemäß einem der Ansprüche 1 bis 6, wobei die Lichtquelle ausgebildet ist, Licht intermittierend unter Bedingungen einer Bestrahlungsstärke von 15 W/cm² oder höher und 50 W/cm² oder niedriger und einer Bestrahlungszeit von 500 ms oder länger und 1000 ms oder kürzer zu emittieren.

## Revendications

1. Dispositif luminescent d'épilation comprenant :
une source de lumière (10) configurée pour émettre de la lumière ayant une longueur d'onde de 400 nm ou plus longue et de 1 200nm ou plus courte ;
une unité de refroidissement de la peau (20) qui fait face à la source de lumière, est configurée pour transmettre la lumière émise depuis la source de lumière, et pour refroidir la peau lors de l'entrée en contact avec la peau ; et
un commutateur à bouton poussoir (30) qui inclut une unité de pression (32) entourant une périphérie externe de la source de lumière et l'unité de refroidissement de la peau, dans lequel
lorsque l'unité de pression n'est pas pressée, l'unité de pression fait saillie depuis une surface de l'unité de refroidissement de la peau vers un côté opposé de la source de lumière par rapport à l'unité de refroidissement de la peau, la surface de l'unité de refroidissement de la peau étant une surface qui doit être portée en contact avec la peau,
lorsque l'unité de pression (32) est pressée par la peau, une surface de l'unité de pression est pressée par la peau pour être déplacée vers la source de lumière par rapport à l'unité de refroidissement de la peau, et
le commutateur à bouton poussoir commute la source de lumière pour émettre la lumière et ne pas émettre la lumière pour amener la source de lumière à continuer d'émettre de la lumière au moins pour une partie du temps tandis que l'unité de pression est pressée, et à ne pas émettre de lumière lorsque l'unité de pression n'est pas pressée.

2. Dispositif luminescent d'épilation selon la revendication 1, configuré pour irradier la peau avec de la lumière depuis la source de lumière après que la peau entre en contact avec la surface de l'unité de refroidissement de la peau.

3. Dispositif luminescent d'épilation selon la revendication 1 ou 2, dans lequel la source de lumière inclut une diode luminescente (DEL).

4. Dispositif luminescent d'épilation selon l'une quelconque des revendications 1 à 3, dans lequel l'unité de refroidissement de la peau est configurée pour être refroidie à -5°C ou plus et 35°C ou moins.

5. Dispositif luminescent d'épilation selon l'une quelconque des revendications 1 à 4, dans lequel une transmittance totale de la lumière de l'unité de refroidissement de la peau est de 80 % ou plus.

6. Dispositif luminescent d'épilation selon l'une quelconque des revendications 1 à 5, dans lequel l'unité de refroidissement de la peau inclut au moins l'un sélectionné dans le groupe constitué de Al₂O₃, ZnO, ZrO₂, MgO, GaN, AIN, et du diamant.

7. Dispositif luminescent d'épilation selon l'une quelconque des revendications 1 à 6, dans lequel la source de lumière est configurée pour émettre de la lumière de manière intermittente sous des conditions d'éclairement énergétique de 15 W/cm² ou plus et de 50 W/cm² ou moins et d'un temps d'irradiation de 500 ms ou plus long et de 1 000 ms ou plus court.
